# EUROPEAN PATENT APPLICATION

(11) **EP 4 282 951 A1**
(43) Date of publication of application: **29.11.2023**
(21) Application number: 22305777.9
(22) Date of filing: 25.05.2022
(51) Int. Cl.: C12N 5/071, A61K 35/39

(54) **METHOD FOR OBTAINING EXTRACELLULAR VESICLES FROM BETA CELLS**

(71) Applicant: École Nationale Vétérinaire, Agroalimentaire et de l'Alimentation, Nantes-Atlantique (ONIRIS), 44300 Nantes (FR)
(72) Inventor: BACH, Jean-Marie, 44470 Carquefou (FR); BOSCH, Steffi, 44470 Mauves sur Loire (FR); DE BEAUREPAIRE, Laurence, 44000 Nantes (FR); MOSSER, Mathilde, 44300 Nantes (FR)
(74) Representative: Plasseraud IP

(57) **Abstract**

The present invention relates to a method for obtaining extracellular vesicles from beta cells comprising at least a step (i) of culturing beta cells in the form of beta cell aggregates in suspension in a cell culture medium under stirring conditions to obtain extracellular vesicles from the beta cells. The invention also relates to the extracellular vesicles, in particular the small extracellular vesicles, obtainable by the method of the invention.

## Description

### Background

Type 1 diabetes melliltus (T1D) is a chronic disease occurring in young children or teenagers that results from the autoimmune destruction of insulin-secreting beta cells in the pancreatic endocrine islets. The disease affects more than 20 million people worldwide with a very high annual economic cost.

While daily insulin therapy is effective in treating the hyperglycemia caused by the disease, it is compulsory, and multiple complications can develop in the short term (hypoglycemia) and long term (renal, cardiac and ocular). A consensus is emerging today on the need to develop combination therapies allowing to reprogram autoimmunity to a state of tolerance, maintenance of pancreatic beta function and the prevention of certain pathogenic mechanisms.

Induction of protective immune memory requires the presentation of the specific antigen(s) in the presence of appropriate tolerogenic signals. Major challenges concern the selection of the antigens, their formulation, dose and route of administration. In T1D patients, diversification of the autoreactive immune lymphocyte repertoire during the asymptotic phase preceding diagnosis, jeopardizes mono-antigenic approaches.

The use of small extracellular vesicles (sEV) of healthy pancreatic beta cells opens up new avenue for the multi-antigenic approaches for the treatment of T1D. sEV of healthy pancreatic beta cells present a cocktail of beta-antigen in association with dominant and evolutionary conserved immune-regulatory signals. The use of allogeneic sEV for therapy does not constitute a limit as the transfer of allogeneic EVs in animals does neither induce toxicity nor inflammation, and, in humans, the transfer of allogeneic EV from mesenchymal stem cells does not engender adverse events, or only very faintly. Murine and human allogeneic beta-sEV have been shown to improve the function, viability and protection of pancreatic beta cells. However, the *in vitro* production of sEV derived from beta cells for therapeutic use faces many difficulties. EV are complex polydisperse bioproducts that mirror the state of the parental cell they are derived from. In this context, there is accumulating evidence that the culture conditions are decisive for the properties of the sEV produced. Several publication show that exposure to stress in culture mirroring pathological conditions such inflammation and hypoxia (Giri, et al., 2020) or increased shearing forces in blood vessels (Piffoux, et al., 2019) modifies the release of the sEV and their phenotype. In addition, sEV are currently mostly produced at laboratory scale in tissue plates, which offer a limited surface and require repeated sub-culturing. In order to observe a therapeutic effect, studies in mice use large amounts of EV, up to 200 µg or 10¹⁰ particles per animal (Varderidou-Minasian et al., 2020). Similarly, 10⁹ to 10¹² particles were administered per patient in recent clinical studies (Herrman et al., 2021). Based on the average yield of 2.5 µg EV protein/10⁶ producer cells, calculated from the results of 54 publications (Gudbergsson et al., 2016) and in line with the studies of the inventors on the beta cells EV (Giri et al., 2020 ; Bosch, et al., 2016), the treatment of a mouse would require the production of 8 × 10⁷ cells. The treatment of a 70 kg human would hence need the production of EV by 2.6 × 10¹⁰ - 2.6 × 10¹³ cells. Future clinical applications of pancreatic beta cells will thus require high-yield culture protocols.

Thus, there is a need in the art for a simple, efficient, and scalable method for obtaining extracellular vesicles from culturing mature cells such as beta cells allowing to preserve the unstressed phenotype of the beta cells during culture, and thus that of the extracellular vesicles obtained therefrom.

### Brief description

In a first object, the present invention relates to a method for obtaining extracellular vesicles from beta cells comprising at least a step (i) of culturing beta cells in the form of beta cell aggregates in suspension in a cell culture medium under stirring conditions to obtain extracellular vesicles from the beta cells.

Preferably, the beta cell aggregates produce and secrete extracellular vesicles in the cell culture medium.

Preferably, the cell culture medium is a serum-free medium.

Step (i) is preferably performed during a period allowing to maintain a cell viability higher than 90%, for example between 2 hours and 50 hours, preferably between 4 and 24 hours.

Step (i) is preferably performed at a stirring speed of between 60 and 160 rpm, preferably between 90 and 120 rpm.

The beta cell aggregates are preferably pseudo-islets.

Step (i) may be preceded by a step (i₀) of forming the pseudo-islets comprising seeding a cell culture medium with isolated beta cells.

Preferably, the cell culture medium is seeded at a cell density of between 0.3×10⁶ and 10⁷ cells per mL of cell culture medium.

The beta cells may be pancreatic continuous cell line derived beta cells, stem-cell derived beta cells or primary beta cells.

The pancreatic continuous cell line is preferably selected from 1.4E7, PANC-1, 1.1B4, 1.1E7 or EndoC-βH1 cell lines.

Step (i) and/or step (i₀) is preferably performed in a stirred tank bioreactor.

Preferably, in step (i) culturing the beta cells aggregates is not associated to an increase of expression of markers associated with beta cells stress such as CHOP, spliced XBP1, ATF4, ATF3, NF-kB or GRP94.

The method of the invention may further comprises a step (ii) of isolating small extracellular vesicles having a size ranging between 20 and 150 nm from the extracellular vesicles obtained at the end of step (i).

In an embodiment, step (ii) comprises the steps of:
(iia) a primary clarification by centrifugation or depth filtration, preferably by normal flow filtration (NFF),
(iib) a concentration by ultracentrifugation or Tangential Flow Filtration (TFF), and
(iic) a filtration by low pressure chromatography, preferably by size exclusion chromatography (SEC) or by bind and elute-size exclusion low pressure chromatography (BE-SEC).

In a second object, the invention relates to the extracellular vesicles, in particular the small extracellular vesicles having a size ranging between 20 and 150 nm, obtainable by the method according to the invention. The small extracellular vesicles are preferably obtained by the step (ii) as described above of isolating small extracellular vesicles having a size ranging between 20 and 150 nm from the extracellular vesicles obtained at the end of step (i).

### Detailed description

As shown in the experimental part below, the inventors have found that the cell viability of beta cells cultured as aggregates in suspension under stirring conditions was significantly decreased, but that, quite surprisingly, there was no increase of stress markers expression in the beta cells remaining in the culture, meaning that these beta cells and the extracellular vesicles obtained therefrom preserved an unstressed phenotype. Further optimization of the culture conditions allowed to sustain a high cell viability and beta cell function, while minimizing the expression of cellular stress markers. Besides, they also found that, surprisingly enough in view of the above-mentioned decrease of cell viability, the beta cells cultured as aggregates under stirring conditions in fact produced significantly more extracellular vesicles than beta cells as aggregates under static conditions. These finding are of paramount importance since stirred systems can be performed at large scale.

A first object of the invention is hence a method for obtaining extracellular vesicles from beta cells comprising at least a step (i) of culturing beta cells in the form of beta cell aggregates in suspension in a cell culture medium under stirring conditions to obtain extracellular vesicles from the beta cells.

### Step (i)

In the method of the present invention, the beta cell aggregates produce and secrete the extracellular vesicles in the cell culture medium during the culture of step (i). In other terms, the culture obtained at the end of step (i) comprises beta cell aggregates and extracellular vesicles in suspension in the cell culture medium.

In the present invention, "in suspension" means a state wherein the cells are submerged in the cell culture medium, typically in a dynamic state in which the cells are in motion within the cell culture medium, and the cell culture medium moves around the cells. In contrast, "static" means a state in which cells do not move relative to the environment in which the cell culture is being performed.

### Beta cells and beta cell aggregates

In the present invention, "beta cell aggregate" herein abbreviated "aggregate" means an assembly of beta cells wherein the cells are grouped together. It encompasses pseudo-islets and primary islets. "Pseudo-islets" refers to aggregates formed from isolated beta cells cultured in suspension to spontaneously form aggregates. "Primary islets" refers to cell aggregates harvested from an explant material originating from the pancreas, in particular from pancreatic islets.

"Pancreatic islets", also known as islets of Langerhans, refers to the regions of the pancreas that contain the endocrine, i.e. the hormone-producing cells. In particular, a pancreatic islet is constituted by a thin fibrous connective tissue capsule surrounding the hormone-producing cells. The term "pancreatic islet" as used herein also comprises superstructures of pancreatic islets, also called islet clusters.

In other terms, "pseudo-islet" refers to structures that preferably contain only beta cells in contrast to "primary islets" that refers to structures that may contain other hormone producing cells such as alpha and delta cells.

In an embodiment, the number of cells per aggregate is comprised between 10 and 2000.

In an embodiment, the aggregates have a size in diameter comprised between 30 and 200 µm. The size in diameter of the aggregates may for example be comprised between 30 and 150 µm, for example between 30 and 100 µm.

In the present invention, "beta cells" or "insulin-secreting beta cells" means cells which secrete insulin when glucose-stimulated. The beta cells may be of human or animal origin, such as a mouse, a dog, a cat or a horse, preferably are human.

In an embodiment, the beta cells express at least one marker characteristic of beta cell function selected from the group consisting of: PDX1, Nkxx6.1, INS, GLUT1, GLUT2, MAFA, PAX4, NGN3, glucokinase, PC1/3 and PC2. In particular, PC2 transcript has been reported in 1.4E7 cells (McCluskey et al., 2011) and in PANC1 cells (Yuan et al., 2013).

Such markers can be monitored by any technique known by the skilled artisan, for example by transcriptomic analysis by real-time RT-PCR.

In an embodiment, the beta cells are obtained from a beta cell line, in particular a continuous cell lines derived from pancreas, preferably derived from pancreatic islets.

Examples of human pancreatic continuous cell lines that are commercially available include but are not limited to 1.4E7 (European Collection of Authenticated Cell Cultures (ECACC) catalogue No 10070102), PANC-1 (ECACC catalogue no. 87092802), 1.1B4 (ECACC catalogue No. 10012801), 1.1E7 (ECACC catalogue no. 10070101) or EndoC-βH1 (Ravassard et al., 2011). Examples of murine pancreatic continuous beta cell lines include MIN6.

However, the present invention is no limited to such source of beta cells. For example, the beta cells may be stem cell-derived beta cells or primary beta cells.

In an embodiment, the beta cells are stem cell-derived beta cells (SC-beta cells). Such SC-beta cells may be prepared by differentiating stem cells, in particular pluripotent stem cells e.g. mesenchymal stem cells or induced pluripotent stem cells (iPSCs), to a more mature, differentiated state, capable of producing insulin when glucose stimulated. The skilled person knows how to prepare such SC-beta cells, for example by following the *in vitro* differentiation protocols as described in Hogrebe et al., 2021.

In an embodiment, the beta cells are primary beta cells. Such primary beta cells may be prepared by culturing beta cells harvested from an explant material originating from the pancreas in particular from pancreatic islets.

### Cell culture medium

The method of the present invention can in principle be performed in any type of cell culture medium suitable for the culturing of beta cells.

In an embodiment, the cell culture medium comprises a nutrient medium.

A "nutrient medium" herein designates any culture medium which favors the maintenance and/or growth of cells which comprise at least part of the following elements: a carbohydrate source, salts and/or amino acids and/or vitamins and/or lipids and/or detergents and/or buffers and/or growth factors and/or hormones and/or cytokines and/or trace elements. Examples of carbohydrate sources include glucose, fructose, galactose and pyruvate. Examples of salts include magnesium salts, for example MgCl₂ .6H2 O, MgSO₄ and MgSO₄ .7H₂O iron salts, for example FeSO₄ .7H₂O, potassium salts, for example KH₂PO₄ , KCI; sodium salts, for example NaH2 PO4, Na₂HPO₄ and calcium salts, for example CaCl₂ .2H₂O. Examples of amino acids include all known proteinogenic amino acids, for example histidine, glutamine, threonine, serine, methionine. Examples of vitamins include: ascorbate, biotin, choline, myoinositol, D-panthothenate, riboflavin. Examples of lipids include fatty acids, for example linoleic acid and oleic acid; Examples of detergents include Tween^{®} 80 and Pluronic^{®} F68. Example of buffers include HEPES and Na₂CO3. Examples of growth factors/hormones/cytokines include IGF (insulin-like growth factor), hydrocortisone and (recombinant) insulin. Examples of trace elements include Zn, Mg and Se.

Non-limitative examples of such a nutrient medium are Dublecco's Modified Essential Media (DMEM), Eagle's Modified Essential Media (EMEM), Advanced RPMI (Roswell Park Memorial Institute) 1640 (RPMI-1640), and Iscove's Media.

In an embodiment, the cell culture medium may also comprise beta cells differentiation components to increase beta cells differentiation and/or function. These beta cells differentiation components preferably comprise at least one component selected from the group consisting of retinoic acid, latrunculin A, SANT1 (Yung et al, 2019), nicotinamide, exendin-4 (Hassouna et al, 2018).

To produce extracellular vesicles from beta cells according to the method of the invention, in particular extracellular vesicles that are intended to be used as an active ingredient in pharmaceutical preparations, serum-free cell culture media are preferred to media containing an animal-derived serum. The reason for this is that serum may be contaminated with EVs, viruses, or may present the risk of prionic infections and can create an obstacle in the further purification of the extracellular vesicles from the cell culture.

Therefore, the method of the invention is preferably performed in a cell culture medium that is serum-free i.e. that does not comprise animal-derived serum such as fetal calf serum (FCS). Since compounds from a mammalian source also present an infection risk, the cell culture medium preferably does not comprise serum or components from a mammalian source. More preferably the cell culture medium does not comprise serum or components from an animal, including human.

In an embodiment, the serum-free cell culture medium comprises a nutrient medium supplemented with serum replacement components. These serum replacement components preferably comprise at least part of the following elements: carbon sources, inorganic salts, amino acids and proteins. In an embodiment, the serum replacement components comprise at least one of transferrin, insulin, selenium, albumin, ethanolamine, Pluronic^{®}F-68 (Cas number: 9003-11-6), or hydrolysates from vegetable such as rapeseed, soybean, chickpea or from yeast. In an embodiment, the protein content of the serum-free cell culture medium is comprised between 5 and 20 µg/mL, preferably between 5 and 20 µg/mL, preferably is of about 15 µg/mL the protein being preferably selected from transferrin, insulin or a mixture thereof.

Examples of serum-free cell culture medium that can be used include commercially available media, such as for instance Dublecco's Modified Essential Media (DMEM), Eagle's Modified Essential Media (EMEM), Advanced RPMI (Roswell Park Memorial Institute) 1640 (RPMI-1640), and Iscove's Media, CMRL islet culture media supplemented with zlslet^{™} Factor Supplement, MSC NutriStem^{®} XF Medium, Serum Free Media (Gibco) or Opti-Mem I^{®} (commercialized under the reference 31985062 by ThermoFischer Scientific).

In an embodiment, the cell culture medium comprises less than 4.5 g/L of glucose. This can advantageously increase the insulin content embarked in EV obtained from the beta cells.

### Culture conditions

As mentioned above, the inventors have found that culturing beta cells in suspension in stirring conditions decreases beta cells viability during the phase of aggregate formation. However, they found that fine-tuning of culture conditions such as stirring speed and culture duration, as well as the initial cell density when the process is performed initiating the culture from isolated beta cells as detailed below, allows to preserve an unstressed phenotype of the cultured beta cells during the phase of EV production while keeping a high EV production.

In the present invention, "unstressed phenotype", relative to the beta cells, refers to a beta cell phenotype which is not associated to an increase of expression of markers associated with beta cells stress, in particular endoplasmic reticulum stress markers, during the culturing.

For example, beta cells stress markers at the transcript level may be selected from the group consisting of: C/EBP homologous protein (CHOP), spliced XBP1, ATF4, ATF3, nuclear factor kappa-light-enhancer of activated B cells (NF-kB) and glucose reactive protein (GRP94). At the protein level, the degree of phosphorylation of certain effectors of the UPR pathway such as elF2a can also be used. In an embodiment, the method of the invention allows to preserve the unstressed phenotype of the beta cells during the culturing, and in particular is not associated to an increase of expression of beta cell stress markers selected from CHOP, spliced XBP1, ATF4, ATF3, NF-kB or GRP94 in said beta cells.

Of course, when performing the method according to the invention, the skilled artisan can monitor the expression of various stress markers associated with beta cells stress, for example by checking their expression or the fact that they are not expressed anymore, and/or by quantitatively measuring their expression level. Any technique known in the art can be used to this aim, for example quantitative RT-PCR, Northern blot, Western blot and immunoassays.

The skilled artisan can also monitor the viability of the beta cells during the culturing step by methods known in the art, typically by measuring the concentration of viable cells (preferably after trypsination of the aggregates, e.g. when the cells are under the form of pseudo-islets) and blue trypan staining and/or the number of dead and lysed cells by measuring LDH.

In the present invention, otherwise stated differently, the cell viability is calculated as the percentage of trypan blue negative viable cells out of total cells.

The inventors believe that monitoring the beta cells stress markers is an important parameter to rely on to adapt the culture conditions such as the stirring speed and/or the duration of culture and the initial cell seeding density as mentioned below, particularly when scaling up the method of the invention, which implies to modulate parameters such as the size of the reactor, the type of cell culture medium or the type of cell line used.

### Duration of culture and speed of stirring

In an embodiment, step (i) is performed with a duration of culture and a stirring speed allowing to maximize cell viability while preserving the beta cells unstressed phenotype.

In an embodiment, step (i) is performed during a period of time allowing the beta cells to generate extracellular vesicles. The culture time is preferably adapted to maintain a cell viability higher than 90%. Preferably, the culture of step (i) is performed during at least 2 hours, preferably during a period of time comprised between 2 hours and 50 hours, preferably between 4 and 24 hours.

In an embodiment, step (i) is performed stirring speed of between 60 and 160 rpm, preferably between 90 and 120 rpm.

### Cell seeding density

In an embodiment, the beta cell aggregates are formed from isolated cells beta cells seeded in suspension under stirring conditions. Thus, in an embodiment, step (i) is preceded by a step (i₀) of seeding a cell culture medium with beta cells under the form of isolated e.g. trypsinated beta cells. The cell culture medium is preferably as described above.

In an embodiment, step (i₀) is performed by seeding the cell culture medium with beta cells under the form of isolated cells at a cell density of between 0.3×10⁶ and 10⁷ cells per mL of cell culture medium.

The inventors have found a high correlation between the cell density, the duration of culture, and the production of EV. They have shown that to obtain a high production of EV it is preferable either to seed the beta cells at a low density and to culture the beta cells as aggregates for a long period of time, or to seed the beta cells at high density and to culture the beta cells as aggregates over a short period of time. The latter is preferred in an industrial production setting.

Thus, in an embodiment step (i₀) and step (i) are performed with a seeding density and a duration of culture allowing to maximize the EV production while preserving the beta cells unstressed phenotype. In a particular embodiment, step (i₀) is performed by seeding the cell culture medium at a high cell density, preferably a cell density comprised 0.3×10⁶ and 10⁷, more preferably between 0.5×10⁶ and 5×10⁶, even more preferably between 2.5×10⁶ and 5×10⁶ cells per mL of cell culture medium, and step (i) is performed over a short period of time, preferably less than 24 hours.

### Bioreactor

In an embodiment, step (i) is performed in a bioreactor, preferably in a stirred tank bioreactor.

In the present invention, a "bioreactor" means a system that comprises the beta cell culture which cell culture on its turn comprises the beta cells and the cell culture medium.

Stirred tank bioreactors (SBRs) are well known by the skilled artisan for the culturing of cells. In these reactors, mechanical stirrers, also termed impellers, are used to mix the reactor and distribute heat and material such as oxygen and substrates as well as keeping the cells in suspension. SBRs generally comprise a baffle to increase the friction to the vessel inner wall surface, convert the rotational flow to axial mixing and avoid the formation of a central vortex. The mechanical stirrer is usually a rotating stirrer that can be attached either at the top or the bottom of the bioreactor.

In an embodiment, the SBR allows for culture volumes of between 20 and 2 000 L, preferably of between 100 and 2000 L.

### Type of culture

In an embodiment of the above method, the culture of step (i) is a batch culture or a fed-batch culture, a continuous culture, a repeated fed-batch culture or a perfused culture.

In the present invention, "Batch" means a culture where the feed, i.e. cell culture medium and/or cell culture components such as nutrients or beta cells differentiation components as described above are provided at the beginning of the cultivation and no extra feeding is used from beginning to end of the culture.

"Fed-batch" means a culture where the cells or cells aggregates are cultivated in an initial cell culture medium to which several additions of feed medium and/or of optional of cell culture components are provided during cultivation.

"Continuous culture" means a culture where the cells or cell aggregates are cultivated in an initial cell culture medium to which additional feed cell culture medium and/or cell culture components are continuously added during cultivation. Broth is preferably withdrawn continuously from the bioreactor.

The inventors believe that the in-line control of cell culture medium and/or of cell culture components addition, in particular the in-line control of nutrient addition, shall allow to obtained control of the unstressed phenotype over long times of culture.

"Perfused culture" means a culture, wherein the cells aggregates are retained in the bioreactor while the desired product i.e. the EVs are removed from the bioreactor throughout the run. The inventors believe that perfused culture shall be a very efficient option in particular as reabsorption may restrict EV yields. The extracellular vesicles can easily be separated from the aggregates. In an embodiment, the bioreactor comprises a retention device suitable to retain the aggregates within the bioreactor and to remove the EVs from the bioreactor.

In an embodiment, the perfused culture is a reiterated fed-batch perfused culture, wherein multiple harvests of extracellular vesicles are reiterated from a same pool of beta cell aggregates. In an embodiment, the method of the invention comprises a step (i) as detailed above and further comprises a step (i-1) of collecting the extracellular vesicles from the culture obtained in step (i), a step (i-2) of collecting the beta cell aggregates from the culture obtained in step (i), and a step (i-3) of culturing the beta cell aggregates of step (i-2), wherein the steps (i-1), (i-2) and (i-3) are reiterated.

In that embodiment the method may for example comprise a set of n iterations, wherein each n-th step (i-1) consists in collecting the extracellular vesicles from the culture obtained in the n-1-th step (i-3), each n-th step (i-2) consists in collecting the beta cell aggregates from the culture obtained in the n-1-th step (i-3), and each n-step (i-3) consists in culturing the beta cell aggregates of the n-th step (i-2). The number of iteration n may be comprised between 1 and 100, preferably between 2 and and 50, more preferably between 2 and 20.

In an embodiment, the steps (i-1), (i-2), and (i-3) are reiterated as long as the beta cell aggregates produce EVs.

In another embodiment, the perfused culture is a continuous perfused culture wherein the incoming feed rate matches the rate of removing of harvest i.e. of cell culture medium comprising the extracellular vesicles.

### Extracellular vesicles

The method of the invention allows to obtain extracellular vesicles from the beta cells.

In the present invention, "extracellular vesicles from the beta cells", "extracellular vesicles derived from beta cells" or "extracellular vesicles" designates the structures which start to appear after culturing beta cells according to step (i). These structures are essentially vesicles comprising a membrane that encloses an internal space. These are released by living beta cells by membrane budding. Extracellular vesicles derived from the plasma membrane are called ectosomes and those derived from the endosome compartment are called exosomes. Such extracellular vesicles generally have a smaller diameter than the mature cells from which they are derived. In particular, the ectosomes, that are formed by protrusions that shed of the cytoplasmic membrane have a size typically between 0.1 and 1 µm and exosomes that are generated as intraluminal vesicles by inward-budding of the endosome and can be released after fusion of these multivesicular bodies with the plasma membrane have a size typically between 20 and 130 nm.

The size of the extracellular vesicles obtained at step (i) may range between 20 nm and 1000 nm in diameter.

These extracellular vesicles may comprise various macromolecular cargo either within the internal space, displayed on the external surface of the extracellular vesicle, and/or spanning the membrane. The cargo may comprise nucleic acids, proteins, carbohydrates, lipids, small molecules, and/or combinations thereof.

In an embodiment, the macromolecular cargo of the extracellular vesicles contains at least one protein specific of beta cells i.e. at least one beta-cells specific antigen selected from insulin, IA-2, GLUT2, ZnT8, GAD65, IAPP, and tetraspanine 7.

In an embodiment, the macromolecular cargo of the extracellular vesicles obtained according to the method of the invention have a phenotype mirroring that of the beta cells, i.e. have an unstressed phenotype.

This unstressed phenotype of the extracellular vesicles may be identified and/or monitored indirectly by monitoring the unstressed phenotype of the beta cell aggregates from which they are generated according to the method of the invention, as described above.

Alternatively, the unstressed phenotype of the beta cells extracellular vesicles may be identified and/or monitored directly on the EVs.

In an embodiment, the unstressed phenotype of the EVs can be identified and/or monitored by at least one of:
- checking the preserved membrane integrity, in particular by cryo-EM imaging,
- checking the quantitative expression of stress markers such as CD63/CD81 of the EVs,
- checking the annexin-5 binding of the EVs,
- checking the little or no release of markers of the unfolded protein response (UPR) signaling pathway by the EVs, or
- checking the little or no release of stress-specific cytokines by the EVs.

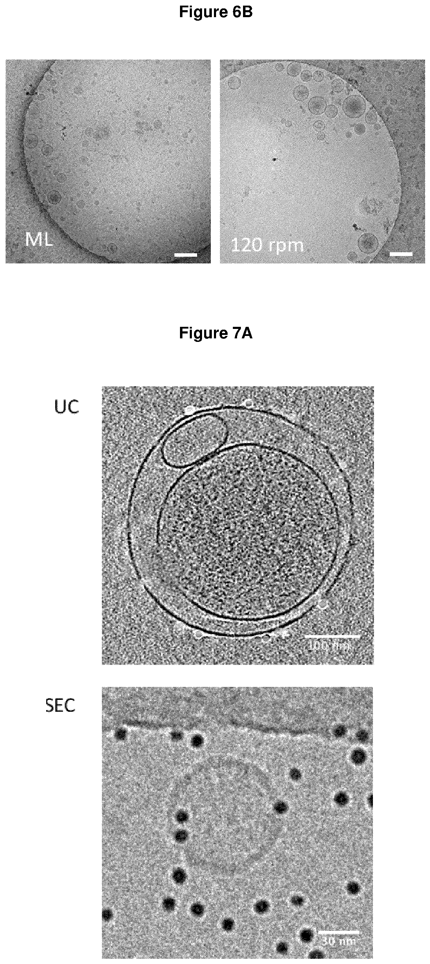

The skilled artisan can easily monitor and/or identify the unstressed phenotype directly on the EVs generated by the method of the invention using cryo-electron microscopy (cryo-EM) imaging. The desired unstressed-specific morphology of EVs as determined by cryo-EM is that of a preserved membrane integrity, that can be identified by the presence membrane vesicles in the absence of non-vesicular material on cryo-EM imaging. An example of preserved membrane integrity specific of unstressed EVs is illustrated on **Figure 6B****,** see the second image from the left.

Expression of CD63 is a characteristic of sEV of endosomal origin while CD81 expression is common to EV of exosomes and ectosomes. Without wanting to be bound by any theory, the inventors believe that shear stress could promote the release of ectosomes from the plasma membrane and hence lower the CD63 to CD81 ratio. In an embodiment, the unstressed phenotype of the EVs obtained by the method according to the invention is identified and/or monitored by evaluating the ratio between CD63 and CD81 expression.

Annexin V binding allows to detect phosphatidyl-serine (PtdSer) exposure on en EV surface. PtdSer is known to be a dominant immune-regulatory signal. Apoptosis triggers PtdSer exposure on the outer leaflet of the plasma membrane and hence on ectosomes derived thereof. Without wanting to be bound by any theory, the inventors believe that shear stress could further promotes PtdSer exposure on EV independently of the apoptosis pathway. In an embodiment, the unstressed phenotype of the EVs obtained by the method according to the invention is monitored and/or identified by evaluating their level of Annexin V binding.

The presence of UPR signaling pathway proteins in sEV from stressed beta cells has been described by Cianciaruso et al., 2017. In an embodiment, the unstressed phenotype of the EVs obtained by the method according to the invention is monitored and/or identified by checking whether the extracellular vesicles contain and/or export little or no of at least one marker of the UPR signaling pathway, for example contain and/or export little or no of at least one marker of the protein immunostimulatory chaperones calreticulin, Gp96, and ORP150. In an embodiment, the unstressed phenotype of the EVs obtained by the method according to the invention is monitored and/or identified by checking whether the extracellular vesicles contain and/or export little or no stress-specific cytokine, in particular little or no of at least one of the particular pro-inflammatory chemokines selected from CXCL10 or MCP1.

### Step (ii)

Of course, when performing the methods according to the present invention, the skilled artisan can isolate subsets of the extracellular vesicles obtained at the end of step (i) based on for example their size, density, biochemical parameters, or a combination thereof.

In an embodiment, the method of the invention further comprises a step (ii) of isolating extracellular vesicles from the culture obtained at the end of step (i) based on their size.

Small extracellular vesicles are particularly targeted for their ease of use in therapy, as they are usually DNA-free and more stable.

In the present invention, "small extracellular vesicles" (abbreviated "sEV) means a subset of extracellular vesicles derived from beta cells having a size ranging between 20 and 250 nm, more preferably between 30 and 150 nm in diameter. In an embodiment, the sEV are DNA-free.

In an embodiment, the method further comprises a step (ii) of separating small extracellular vesicles having a size ranging between 20 and 150 nm, more preferably between 30 and 150 nm in diameter from the culture of step (i).

The sEV may be separated by any techniques known in the art, such as successive centrifugation steps at increasing speed, e.g. of 300 g, 2 000 g and then 10 000 g, followed by an ultracentrifugation (UC) step to pellet the EVs, e.g. at 100 000 g.

However, it is known that the UC step may damage the vesicles, leading to aggregation and fusion and a low sEV recovery yield. Furthermore, the workflow mentioned above does not suit large-scale production of sEV because of the manual work required, the duration of the procedure, and cost of equipment.

As exemplified in the experimental part below the inventors have developed a process allowing to separate the sEVs while solving the problems encountered with the techniques known in the art, according to which the step (ii) can be performed by a process comprising the steps of:
(iia) a primary clarification by centrifugation or depth filtration, preferably by normal flow filtration (NFF),
(iib) a concentration by ultracentrifugation or Tangential Flow Filtration (TFF), and
(iic) a filtration by low pressure chromatography, preferably by size exclusion chromatography (SEC) or by bind and elute-size exclusion low pressure chromatography (BE-SEC)

The Tangential Flow Filtration may be performed with a 300 kDa MWCO.

The filtration by low pressure chromatography using SEC or a bind and elute-size exclusion low pressure chromatography (BE-SEC) allows to obtain a similar purity ratio than for the UF-SEC process.

The BE-SEC enable on-line measurement of DO and conductivity making the process more reproducible than separation on columns driven only by gravity as for SEC.

The very high recovery yields of particles per cell obtained by this separating process particularly adapted to a scalable automated purification process, in particular when combining TFF and BE-SEC methods.

Furthermore, it is preferred to implement filtration techniques rather than centrifugation for primary clarification. There are several types of depth filters available to accommodate process development at commercial manufacturing scales. They are often multilayered, with a nominal size of 0.1-60 µm. The combination of depth filtration, tangential flow filtration and low-pressure chromatography seems suitable for the automated, scalable and consistent downstream processing of sEV isolation from conditioned culture medium.

In an embodiment, step (ii) is preceded by a step (ii₀) of extensive washing of the cells. This is particularly useful when in step (i) the cells are cultured using a cell culture medium containing animal components such as serum.

### Second object

A second object relates to the extracellular vesicles, in particular the small extracellular vesicles obtainable or obtained by the method according of the invention.

The extracellular vesicles obtainable or obtained by the method according of the invention, in particular the small extracellular vesicles, preferably show an unstressed phenotype as described above. In particular, this unstressed phenotype may be monitored and/or identified by at least one of :
- checking the preserved membrane integrity, in particular by cryo-EM imaging,
- checking the quantitative expression of stress markers such as CD63/CD81 of the EVs,
- checking annexin-5 binding of the EVs,
- checking the little or no release of markers of the unfolded protein response (UPR) signaling pathway by the EVs, or
- checking the little or no release of stress-specific cytokines by the EVs,
as detailed above.

The extracellular vesicles obtainable or obtained by the method according of the invention, in particular the small extracellular vesicles, preferably have macromolecular cargo as described above, and in particular preferably contain at least one beta-cells specific antigen selected from insulin, IA-2, GLUT2, ZnT8, GAD65, IAPP, and tetraspanine 7.

The extracellular vesicles obtainable or obtained by the method according of the invention obtainable or obtained by the method according of the invention contain little or no DNA, preferably are DNA free.

### Legend of the Figures

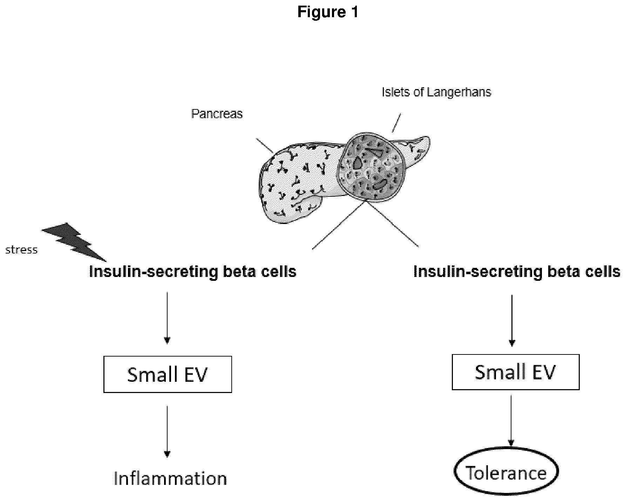

**Figure 1** is as schematic diagram illustrating that when small extracellular vesicles are derived from stressed insulin-secreting beta cells induce inflammation, whereas the small extracellular vesicles derived from unstressed insulin-secreting beta cells induce tolerance.

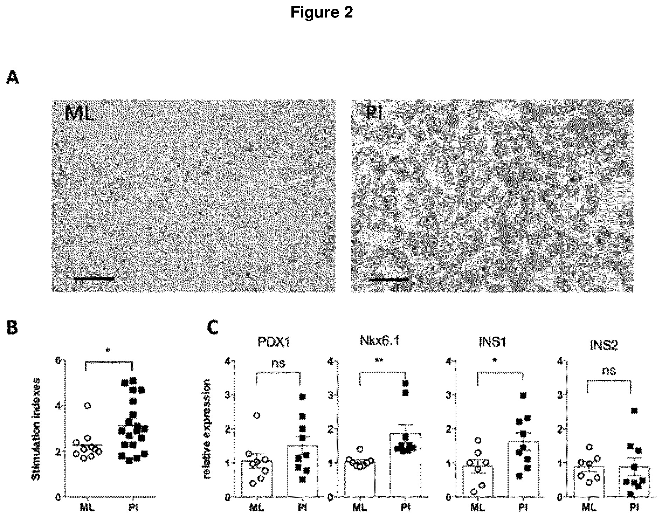

**Figure 2** illustrates that the 3D culture of MIN6 beta cell line promotes beta cell function. **(****Figure 2A****)** MIN6 beta cells were cultured as monolayers (ML) in T-flasks or as pseudo-islets (PI) in spinners. Representative microscope images; scale bar: 200 µm. **(****Figure 2B****)** Stimulation indexes of insulin secretion in response to glucose stimulation **(****Figure 2C****)** Transcriptomic analysis of beta cell function by real-time RT-PCR. Results are plotted as individual values and mean from 7-19 independent experiments. *p<0.05, **p<0.01 (unpaired non-parametric t- test)

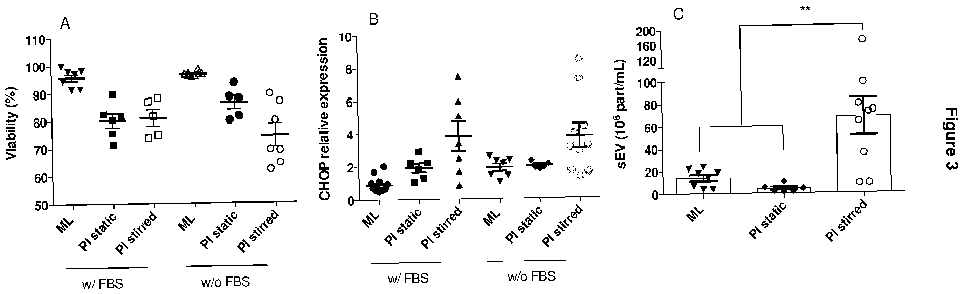

**Figure 3** illustrates that the 3D culture of the MIN6 beta cell line in stirred systems increases cell stress and sEV production. MIN6 beta cells were cultured as monolayers (ML) in T-flasks or as pseudo-islets (PI) in petri dishes (static) and in spinners (stirred) for three days in medium w/FBS. Then, medium was changed and MIN6 cells were cultured as ML, PI static or PI stirred either in medium w/FBS or w/o FBS for 24h at a cell concentration of 2.5 10⁶ cells/mL. **(****Figure 3A****)** Cell viability was assessed by measuring the total of viable cells after trypsin and blue trypan staining and of dead cells by measuring the LDH activity in supernatant. **(****Figure 3B****)** Relative CHOP expression compared to MIN6 cells grown in ML w/FBS. **(****Figure 3C****)** The particle size distribution and concentration of sEV isolated from culture supernatants in medium w/o FBS was determined by TRPS analysis. **p<0.01 (unpaired non-parametric t- test).

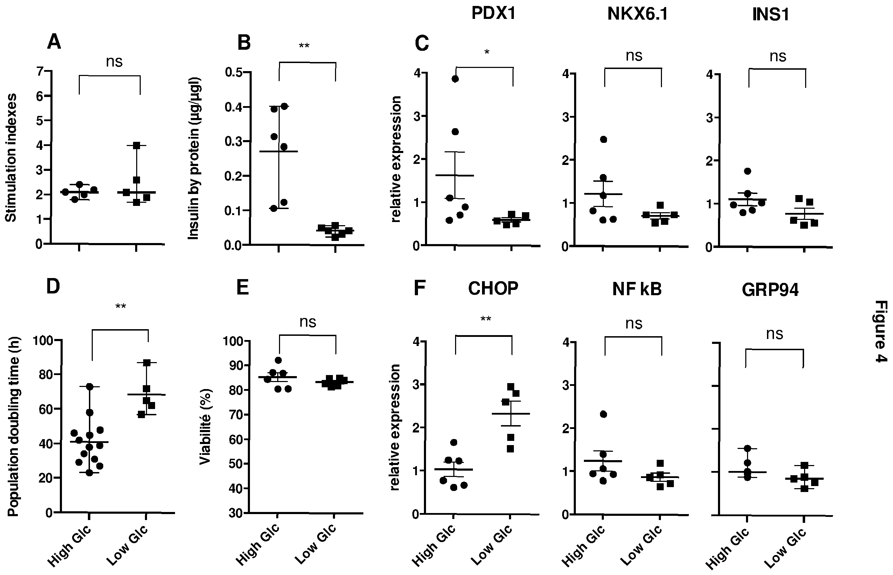

**Figure 4** illustrates the influence of glucose concentration on MIN6 cell phenotype during the amplification step in monolayer culture. MIN6 cells were cultured in DMEM + 10% FBS at high [3.5 - 4.5 g/L] or low [1.0 - 1.5 g/L] glucose concentration. Glucose concentration was daily-assessed (Bioprofil) and adjusted whenever required to reach the targeted ranges. The cells were grown for one week to determine the population doubling time. Beta cell viability and function analysis was performed on day 3. **(****Figure 4A****)** Stimulation index (ratio of high glucose plus theophylline stimulation over basal insulin secretion release) **(****Figure 4B****)** intracellular insulin per total protein content (µg insulin/µg protein). **(****Figure 4C****)** Relative quantitative RT-PCR analysis of expression of beta cell transcription factors pancreatic and duodenal homeobox 1 (PDX1), NKX6 homeobox 1 (NKX6.1) and insulin 1 (INS1). **(****Figure 4D****)** Population doubling time (hour) calculated during the exponential growth phase. **(****Figure 4E****)** Cell viability calculated as the percentage of trypan blue negative viable cells out of total cells. Dead and lysed cells in cell supernatants were estimated by the LDH assay. (F) Relative quantitative RT-PCR expression analysis of C/EBP homologous protein (CHOP), nuclear factor kappa-light-enhancer of activated B cells (NF-kB) and glucose reactive protein (GRP94). Results from independent experiments (n=5-12) are expressed as mean ± SEM. *p<0.05, **p<0.01 (unpaired non-parametric t- test).

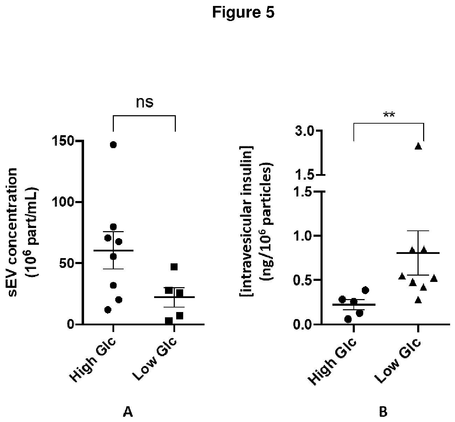

**Figure 5** illustrates the Influence of glucose concentration on MIN6 pseudo-islets culture in medium w/o FBS for sEV production. MIN6 pseudo-islets were cultured in stirred tanks (90 rpm) with Opti-MEM at high [3.5 - 4.5 g/L] or low [2.0 - 2.5 g/L] glucose concentration for one day. **(****Figure 5A****)** The particle size distribution and concentration of sEV isolated from culture supernatants in medium w/o FBS was determined by TRPS analysis. **(****Figure 5B****)** intracellular insulin per 10⁶ particles (ng insulin/10⁶ particles). Results from independent experiments (n=5-8) are expressed as mean ± SEM., **p<0.01 (unpaired non-parametric t-test).

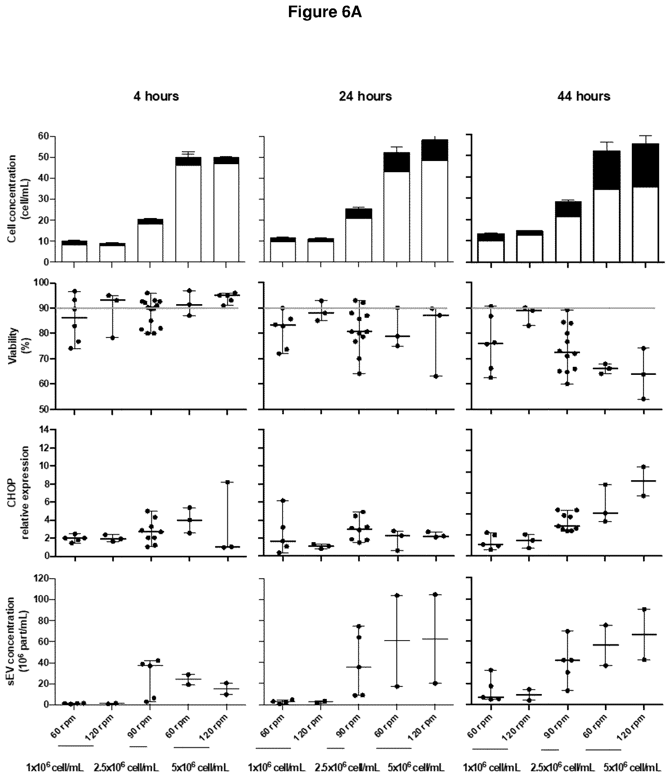

**Figure 6** illustrates the full factorial design plan performed to optimize sEV production by pseudo-islets cultured in stirred bioreactors. MIN6 pseudo-islets were seeded at a concentration of 1.0×10⁶ cell/mL, 2.5×10⁶ cell/mL or 5.0×10⁶ cell/mL and cultured in a medium without FCS in spinners with stirring of 60, 90 or 120 rpm for 4, 24 or 44 hours. The following parameters have been followed: **(****Figure 6A****)** concentration of viable cells (white) and dead cells (black) (10⁵ cell/mL), the viability (%), the relative expression of CHOP compared to PI cultured in spinner in medium w/FBS at 90 rpm, the concentration of sEV (10⁶ particles/mL) as well as **(****Figure 6B****)** the morphology of sEV produced in the absence or presence of stirring at moderate (120rpm) speed using cryo-electron microscopy analysis. Scale bar: 200nm.

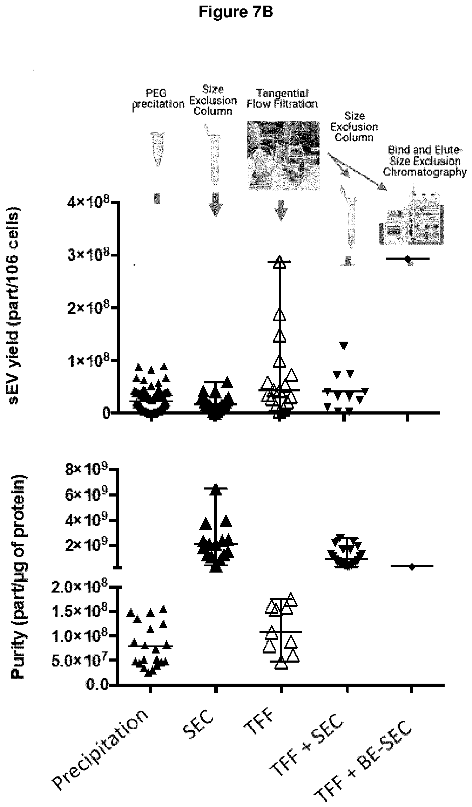

**Figure 7** illustrates the comparison of downstream processes of beta-sEV produced from murine pseudo-islets cultured in stirred tanks. After sequential centrifugation (300 g, 2,000 g and 16,000 g), the beta-sEV were either pelleted using ultracentrifugation (100,000 g, UC) or polymer precipitation (Exoquick) or purified by frontal ultrafiltration with a 100 kDa MWCO (Amicon) followed by size exclusion chromatography (qEVoriginal/35 nm, IZON) (SEC), a Tangential Flow Filtration (KrosFlo^{®}, Repligen) with a 300 kDa MWCO (Spectrum^{®} MidiKross) alone (TFF) or followed by either regular (TFF + SEC) or bind and elute size exclusion chromatography at low pressure (BE-SEC, HiTrap Capto Core 400, GE Healthcare) (GE HealthCare). **(****Figure 7A****)** Cryo-electron microscopy images of beta sEV obtained by UC and SEC purification. Representative images showing a multilamelar (UC) or single sEV with a diameter 82 nm +/- 5 nm (SEC). **(****Figure 7B****)** sEV yield expressed as the number of particles recovered per 10⁶ seeding cells and purity ratio expressed as the number of particles per µg of protein.

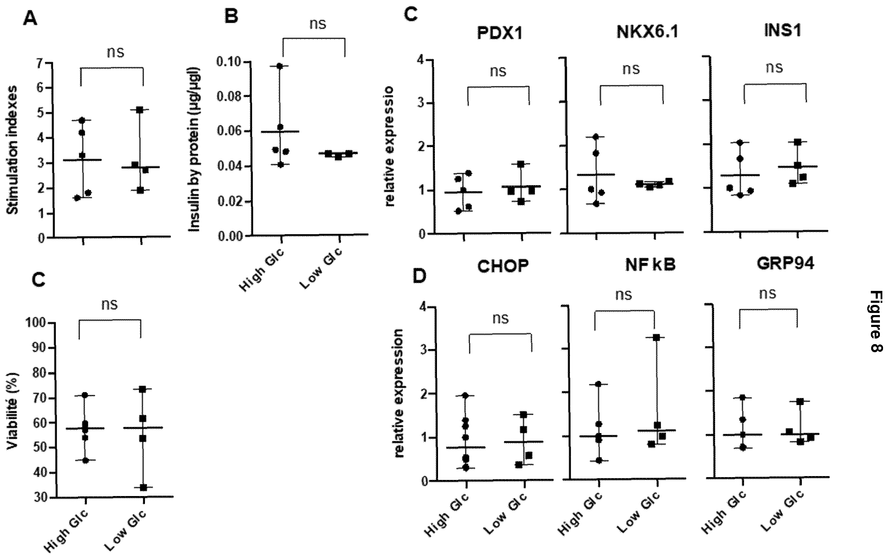

**Figure 8** illustrates the influence of glucose concentration on MIN6 pseudo-islets culture in medium w/FBS. MIN6 cells were cultured for 3 days in stirred tank (90 rpm) with DMEM + 10% FBS with high or low glucose concentrations controlled between 4.5 and 3.5 g/L or 1.5 and 1 g/L respectively. Glucose was daily assessed (Bioprofil) and refreshed as required to reach the targeted ranges. The cells were analyzed at day 3. **(****Figure 8A****)** Stimulation index (ratio of high glucose plus theophylline stimulation over basal insulin secretion release) **(****Figure 8B****)** intracellular insulin content by total protein (µg insulin/µg protein). **(****Figure 8C****)** Relative quantitative RT-PCR expression analysis of pancreatic progenitor transcription factor (PDX1), NKX6.1 (NKX6.1) and insulin (INS1). **(****Figure 8D****)** cell viability calculated as the percentage of trypan blue excluded viable cells by total using dead and lysed estimated by LDH assay in cell supernatant. **(Figure 8E)** Relative quantitative RT-PCR expression analysis of C/EBP homologous protein (CHOP), nuclear factor kappa-light-enhancer of activated B cells (NF kB) and glucose reactive protein 94 (GRP94). Results from independent experiments (n=4-5) are expressed as mean ± SEM. **p<0.01 (unpaired non-parametric t- test).

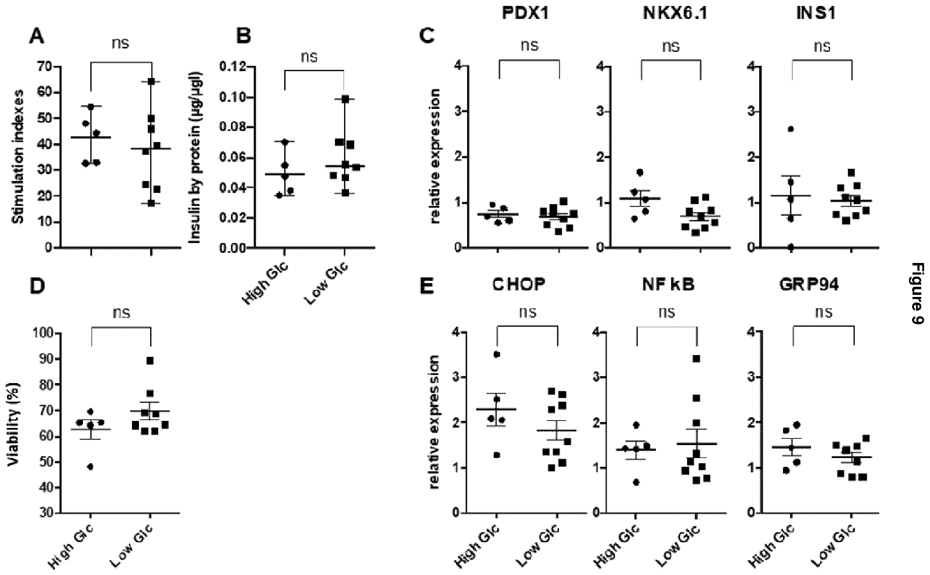

**Figure 9** illustrates the influence of glucose concentration on MIN6 pseudo-islets culture in medium w/o FBS for sEV production step. MIN6 cells were cultured for 1 day in stirred tank (90 rpm) with Optimem with high or low glucose concentrations controlled between 4.5 and 3.5 g/L or 2.5 and 2 g/L respectively. The cells were analyzed at day 1. **(****Figure 9A****)** Stimulation index (ratio of high glucose plus theophylline stimulation over basal insulin secretion release) **(****Figure 9B****)** intracellular insulin content by total protein (µg insulin/µg protein). **(****Figure 9C****)** Relative quantitative RT-PCR expression analysis of pancreatic progenitor transcription factor (PDX1), NKX6.1 (NKX6.1) and insulin (INS1). **(****Figure 9D****)** cell viability calculated as the percentage of trypan blue excluded viable cells by total using dead and lysed estimated by LDH assay in cell supernatant. (E) Relative quantitative RT-PCR expression analysis of C/EBP homologous protein (CHOP), nuclear factor kappa-light-enhancer of activated B cells (NF kB) and glucose reactive protein 94 (GRP94). Results from independent experiments (n=5-9) are expressed as mean ± SEM. **p<0.01 (unpaired non-parametric t- test).

### Examples

The present disclosure is further illustrated by the following examples.

### Example 1: Scalable production of small extracellular vesicles from mature beta cell aggregates for immune therapy for type 1 diabetes: integrating management of cellular stress in upstream processing

### 1.1. MATERIALS AND METHODS

### Cell culture

MIN6 cells (kindly provided by Prof. Jun-ichi Miyazaki, University Medical School, Osaka, Japan) were cultured as previously described (Giri, K. R. et al., 2020. Briefly, for routine expansion, MIN6 cells were grown as adherent cells in tissue culture flasks -T-flask) plated at a density of 1.5 × 10⁵ cells/cm² in DMEM high glucose medium (Life Technologies, Saint Aubin, France) supplemented with 10% FCS (Eurobio, Les Ulis, France) and 20 µM beta-mercaptoethanol (SIGMA, Saint Quentin Fallavier, France). Cultures were regularly assessed for mycoplasma contamination using the MycoAlert TM mycoplasma detection kit (Lonza, Basel, Switzerland). For pseudo-islet formation under static or dynamic conditions, MIN6 cells were seeded in complete medium at a density of 1×10⁶ cells/mL onto non-surface treated petri dishes or spinner (Corning, Sigma Aldrich) at a stirring speed set to 90 rpm (Biomix, 2mag, Deutschland), respectively. Prior to EV production, MIN6 cells were washed twice in PBS w/o Ca2⁺ and Mg²⁺ (Eurobio Scientific, Les Ulis, France) and switched to serum-free OptiMEM (Life Technologies) supplemented with 20µM beta-mercaptoethanol at densities indicated in figure captions. Supernatants containing MIN6 extracellular vesicles were harvested 4h, 24h or 44h later.

### Antibodies & reagents

As a marker of cell death, lactate dehydrogenase activity was measured in culture supernatants using a colorimetric detection kit (Roche, Diagostics, Meylan, France). Serial dilutions of MIN6 cells lysed in 2% Triton X-100 served to construct a standard curve to infer absolute amounts of lysed cells in application of the supplier's recommendations.

### Protein analyses

For protein analyses, cells were lysed in RIPA lysis buffer containing proteases/phosphatases inhibitors (Ozyme, Saint Cyr l'Ecole, France) or in ethanol acid buffer followed by Tris 1M pH 7.5 neutralization. sEV were lysed in 0.1% Triton X-100. Protein concentration was determined by a Bradford protein assay using Coomassie plus assay reagent (Fisher Scientific) following the supplier's recommendations. Optical densities were read on a Fluostar instrument (BMG LABTECH, Champigny sur Marne, France).

### Static glucose-stimulated insulin-secretion assay.

To evaluate beta cell function of MIN6 monolayer (ML) or pseudo-islet (PI), 1E6 cells were pre-incubated in a P6.-well in low glucose medium composed of RPMI supplemented with 2 mM L-glutamine, 2.8 mM glucose and 0.5% bovine albumin for 60 min. at 37 °C, 5% CO₂. Following this period, cells were stimulated at high 20 mM glucose concentration in the presence of 10 mM theophylline for one hour. 1 mL of culture supernatant were then carefully sampled to avoid accidental islet uptake, centrifuged at 200g for 3 min. and stored at -20 °C until analysis. Total mouse insulin secreted by MIN6 cells was quantified by ELISA (Mercodia, Upsala, Sweden).

### Intracellular insulin

MIN6 cells grown as ML or PI were lysed in acidic ethanol buffer for insulin extraction.

### Isolation of beta sEV

EV were collected from MIN6 supernatants using a method combining differential centrifugation, tangential flow filtration and size-exclusion chromatography. Briefly, conditioned culture media were centrifuged immediately after harvest at 300 × *g* 10 min., 2,000 × g, 20 minutes and 16,000 × g, 20 minutes to discard cell debris and large vesicles. The 16,000 supernatants were filtered 0.2µm and sEV isolated using a 300kD MidiKros column (Repligen, Netherlands) on a KrosFlo Research IIi Tangential Flow Filtration instrument (Spectrum Europe, Netherlands). The input flow rate was set to 50 mL/min. resulting in a transmembrane pressure of approximately 300mbars. sEV were concentrated down to 20 mL, followed by four cycles of diafiltration in PBS.

Whenever indicated, sEV were concentrated down to approximately 100 µL on an AMICON MWCO-100 kDa cellulose ultrafiltration unit (Dutscher, Issy-les-Moulineaux, France) and passed through a qEV single size exclusion chromatography column (IZON, Lyon, France). sEV were collected in flow-through fractions two and three. sEV were stored at 4 °C for one to three days or at -80 °C in PBS 25 mM Trehalose.

### Exoquick

For direct quantification of sEV, ExoQuick exosome isolation reagent (Ozyme, Saint Cyr I'Ecole, France) was added to 2.5 - 5.0 mL of crude culture supernatant following the supplier's recommendation.

### Tunable resistive pulse sensing (TRPS)

The size and concentration of sEV were analysed by tunable-resistive pulse sensing using a qNANO instrument and NP100 or NP150 nanopores (IZON, Lyon, France). All samples were diluted in PBS 0.03% Tween-20 electrolyte. After instrument calibration with 110 nm or 210 nm calibration beads (Izon) for UF/TFF or Exoquick purification-based methods, respectively. All samples were analysed with at least two different pressures.

### Transcriptomic analysis

Total RNA was extracted from MIN6 cells using the miRVana extraction reagent (Fisher Scientific). Prior to quantitative real-time PCR analysis, 3µg of RNA were treated with TurboDNase (Fisher Scientific) and reverse transcribed using M-MLV Reverse Transcriptase (Fisher Scientific) and random 15-mers primers (Eurogentec, Angers, France). Mouse markers of beta function and endoplasmic reticulum stress were quantified using primer sequences described earlier (Javeed et al, 2021 ; Oslowski et al, 2011) and Evagreen reagents (Solisbiodyne, Tartu, Estonia) on a CFX96 Touch Real-time PCR detection system (Bio-Rad, Marnes La Coquette, France) in application of the relative standard curve method. Relative quantities of target gene expression were normalised with respect to a normalisation factor calculated as the geometric mean of the sample's housekeeping genes GAPDH and HPRT1 expression.

### Cryo-electron microscopy

For morphological analysis, sEV were processed for cryo-electron microscopy as described earlier (Giri et al., 2020) and analysed on a Tecnai G²T20 sphera electron microscope (FEI company, The Netherlands) equipped with a CCD camera (US4000, Gatan) at 200 kV. Images were reconstructed using the graphical user interface eTomo from the IMOD software package (Mastronarde et al., 1997).

### Experimental design

Screening of the cell culture conditions impacting MIN6 beta cells was performed using a full factorial design 2³⁻¹ **(****Figure 2****).** The influence of the main factors and their first-order interactions were analyzed. The three factors studied and their levels were: culture mode (monolayer/pseudo-islets), agitation (static/ stirred) and medium formulation (with or without FBS). The cultures of stirred monolayer (w/ or w/o FBS) were excluded as there are not relevant conditions of culture. The response variables were (i) cell viability by measuring the concentration of viable cells after trypsination and blue trypan staining and the number of dead and lysed cells by measuring LDH (%) and (ii) relative CHOP expression compared to the reference culture (ML in medium w/ FBS). These response variables were assessed after 24 hours of culture under the different conditions defined by the experimental design (***Table 1***)*.*

### Response Surface Method (RSM)

RSM was used to optimize the beta cells condition of culture to produce sEV from healthy unstressed cells in a scalable process. The objective was to maximize the cell viability, to minimize cellular stress and to maximize sEV production by tuning the stirring, the cell density and the duration of culture. A central composite design (2³ factorial design with 4-star points and four replicates of the central point) was used to fit a second-order polynomial model **(****Figure 3C****).** The model validation was performed by analyzing the lack-of-fit test results, the determination coefficient R² value, and the diagnostic plots. The three factors studied were stirring (60, 90 and 120 rpm), cell density (1, 2.5 and 5 × 10⁶ cell/mL) and duration (4, 24 and 44 hours) of culture. The response variables were cell viability (%), CHO expression (relative expression compared to PI cultured in stirred system at 90 rpm in medium w/ FBS) and sEV production (particle/mL) as defined by the experimental plan (Table 5).

### Statistical analyses.

The experimental design for the screening and the optimization steps were repeated independently at least three times. Analysis of variance (ANOVA), regression analysis, and graphical display of DoE results were performed using the Statgraphics Centurion software 18.1.06. Assessment of the optimal BAP design on NPIs was performed on a minimum of four independent experiments. The significance of differences between groups was evaluated using statistical tests as indicated in figure captions. A p-value <0.05 was considered statistically significant. Graphs' formatting was performed using Graphpad Prism software 8.0.2.

### 3D culture of MIN6 beta cell line promotes beta cell function.

MIN6 beta cells were cultured as monolayers (ML) in T-flasks or as pseudo-islets (PI) in spinners. Representative microscope images; scale bar: 200 µm. The results are shown on **Figure 2: (Figure 2B****)** Stimulation indexes of insulin secretion in response to glucose stimulation **(****Figure 2C****)** Transcriptomic analysis of beta cell function by real-time RT-PCR. Results are plotted as individual values and mean from 7-19 independent experiments. *p<0.05, **p<0.01 (unpaired non-parametric t- test)

### 3D culture of the MIN6 beta cell line in stirred systems increases cell stress and sEV production.

MIN6 beta cells were cultured as monolayers (ML) in T-flasks or as pseudo-islets (PI) in petri dishes (static) and in spinners (stirred) for three days in medium w/FBS. Then, medium was changed and MIN6 cells were cultured as ML, PI static or PI stirred either in medium w/FBS or w/o FBS for 24h at a cell concentration of 2.5 106 cells/mL. The results are shown on **Figure 3** : **(Figure 3A****)** Cell viability was assessed by measuring the total of viable cells after trypsin and blue trypan staining and of dead cells by measuring the LDH activity in supernatant. **(****Figure 3B****)** Relative CHOP expression compared to MIN6 cells grown in ML w/FBS. **(****Figure 3C****)** The particle size distribution and concentration of sEV isolated from culture supernatants in medium w/o FBS was determined by TRPS analysis. **p<0.01 (unpaired non-parametric t- test).

### Influence of glucose concentration on MIN6 cell phenotype during the amplification step in monolayer culture.

MIN6 cells were cultured in DMEM + 10% FBS at high [3.5 - 4.5 g/L] or low [1.0 - 1.5 g/L] glucose concentration. Glucose concentration was daily-assessed (Bioprofil) and adjusted whenever required to reach the targeted ranges. The cells were grown for one week to determine the population doubling time. Beta cell viability and function analysis was performed on day 3. The results are shown on **Figure 4: (Figure 4A****)** Stimulation index (ratio of high glucose plus theophylline stimulation over basal insulin secretion release) **(****Figure 4B****)** intracellular insulin per total protein content (µg insulin/µg protein). (Figure 4C) Relative quantitative RT-PCR analysis of expression of beta cell transcription factors pancreatic and duodenal homeobox 1 (PDX1), NKX6 homeobox 1 (NKX6.1) and insulin 1 (INS1). (Figure 4D) Population doubling time (hour) calculated during the exponential growth phase. (Figure 4E) Cell viability calculated as the percentage of trypan blue negative viable cells out of total cells. Dead and lysed cells in cell supernatants were estimated by the LDH assay. (F) Relative quantitative RT-PCR expression analysis of C/EBP homologous protein (CHOP), nuclear factor kappa-light-enhancer of activated B cells (NF-kB) and glucose reactive protein (GRP94). Results from independent experiments (n=5-12) are expressed as mean ± SEM. *p<0.05, **p<0.01 (unpaired non-parametric t- test).

### Influence of glucose concentration on MIN6 pseudo-islets culture in medium w/o FBS for sEV production

MIN6 pseudo-islets were cultured in stirred tanks (90 rpm) with Opti-MEM at high [3.5 - 4.5 g/L] or low [2.0 - 2.5 g/L] glucose concentration for one day. The results are shown on **Figure 5: (Figure 5A****)** The particle size distribution and concentration of sEV isolated from culture supernatants in medium w/o FBS was determined by TRPS analysis. (Figure 5B) intracellular insulin per 10⁶ particles (ng insulin/10⁶ particles). Results from independent experiments (n=5-8) are expressed as mean ± SEM., **p<0.01 (unpaired non-parametric t-test).

### Full factorial design plan performed to optimize sEV production bv pseudo-islets cultured in stirred bioreactors.

MIN6 pseudo-islets were seeded at a concentration of 1.0×10⁶ cell/mL, 2.5×10⁶ cell/mL or 5.0×10⁶ cell/mL and cultured in a medium without FCS in spinners with stirring of 60, 90 or 120 rpm for 4, 24 or 44 hours. The following parameters have been followed: concentration of viable cells (white) and dead cells (black) (10⁵ cell/mL), the viability (%), the relative expression of CHOP compared to PI cultured in spinner in medium w/FBS at 90 rpm and the morphology and concentration of sEV (10⁶ particles/mL). The results are shown on **Figure 6****.**

### Comparison of downstream processes of beta-sEV produced from murine pseudo-islets cultured in stirred tanks

After sequential centrifugation (300 g, 2,000 g and 16,000 g), the beta-sEV were either pelleted using ultracentrifugation (100,000 g, UC) or polymer precipitation (Exoquick) or purified by frontal ultrafiltration with a 100 kDa MWCO (Amicon) followed by size exclusion chromatography (qEVoriginal/35 nm, IZON) (SEC), a Tangential Flow Filtration (KrosFlo^{®}, Repligen) with a 300 kDa MWCO (Spectrum^{®} MidiKross) alone (TFF) or followed by either regular (TFF + SEC) or bind and elute size exclusion chromatography at low pressure (BE-SEC, HiTrap Capto Core 400, GE Healthcare) (GE HealthCare). The results are shown on Figure 7: **(****Figure 7A****)** Cryo-electron microscopy images of beta sEV obtained by UC and SEC purification. Representative images showing a multilamelar (UC) or single sEV with a diameter 82 nm +/- 5 nm (SEC). **(****Figure 7B****)** sEV yield expressed as the number of particles recovered per 10⁶ seeding cells and purity ratio expressed as the number of particles per µg of protein.

### Influence of glucose concentration on MIN6 pseudo-islets culture in medium w/FBS.

MIN6 cells were cultured for 3 days in stirred tank (90 rpm) with DMEM + 10% FBS with high or low glucose concentrations controlled between 4.5 and 3.5 g/L or 1.5 and 1 g/L respectively. Glucose was daily assessed (Bioprofil) and refreshed as required to reach the targeted ranges. The cells were analyzed at day 3. The results are shown on **Figure 8: (Figure 8A****)** Stimulation index (ratio of high glucose plus theophylline stimulation over basal insulin secretion release) **(****Figure 8B****)** intracellular insulin content by total protein (µg insulin/µg protein). **(****Figure 8C****)** Relative quantitative RT-PCR expression analysis of pancreatic progenitor transcription factor (PDX1), NKX6.1 (NKX6.1) and insulin (INS1). **(****Figure 8D****)** cell viability calculated as the percentage of trypan blue excluded viable cells by total using dead and lysed estimated by LDH assay in cell supernatant. **(Figure 8E)** Relative quantitative RT-PCR expression analysis of C/EBP homologous protein (CHOP), nuclear factor kappa-light-enhancer of activated B cells (NF kB) and glucose reactive protein 94 (GRP94). Results from independent experiments (n=4-5) are expressed as mean ± SEM. **p<0.01 (unpaired non-parametric t- test).

### Influence of glucose concentration on MIN6 pseudo-islets culture in medium w/o FBS for sEV production step.

MIN6 cells were cultured for 1 day in stirred tank (90 rpm) with Optimem with high or low glucose concentrations controlled between 4.5 and 3.5 g/L or 2.5 and 2 g/L respectively. The cells were analyzed at day 1. The results are shown on **Figure 9: (Figure 9A****)** Stimulation index (ratio of high glucose plus theophylline stimulation over basal insulin secretion release) (Figure 9B) intracellular insulin content by total protein (µg insulin/µg protein). **(****Figure 9C****)** Relative quantitative RT-PCR expression analysis of pancreatic progenitor transcription factor (PDX1), NKX6.1 (NKX6.1) and insulin (INS1). **(****Figure 9D****)** cell viability calculated as the percentage of trypan blue excluded viable cells by total using dead and lysed estimated by LDH assay in cell supernatant. (E) Relative quantitative RT-PCR expression analysis of C/EBP homologous protein (CHOP), nuclear factor kappa-light-enhancer of activated B cells (NF kB) and glucose reactive protein 94 (GRP94). Results from independent experiments (n=5-9) are expressed as mean ± SEM. **p<0.01 (unpaired non-parametric t- test).

### 1.1. RESULTS AND DISCUSSION

### Upstream strategy to produce sEV from mature beta cells at large-scale.

Pancreatic beta cells are adherent cells. Cultivated on hydrophobic surface, they spontaneously form pseudo-islets by favoring the formation of intercellular bonds **(****Figure 2A****).** This organization improves their ability to produce insulin in response to glucose stimulation **(****figure 2B****)** by 38 % (p<0.05) and promotes beta cell maturation **(****Figure 2C****)** as shown by increased Nkx6.1 transcription factor (p<0.01) and INS1 expression (p<0.05), while no significant improvement of PDX1 and INS2 have been observed.

In order to assess the feasibility to produce sEV from pseudo-islets in a stirred system, the impact of these conditions on the cell's viability and cellular stress pathways was assessed using a fractional factorial design. In addition, the impact of serum free medium formulation was assessed to mitigate the risk of contamination by EV, viruses, mycoplasma or prions from media supplement of animal origin. The three following factors have been screened: the mode of culture (monolayer/ pseudo-islets), the agitation (static/ stirred systems) and the medium formulation (with/ without FBS) **(Table *1*** and **Figure 3****).** To do so, the cells were first cultured for 3 days in monolayer, static or stirred pseudo-islets in medium w/FBS. Then, the medium was changed and the cells were cultured for 24h in the different conditions and analyzed.

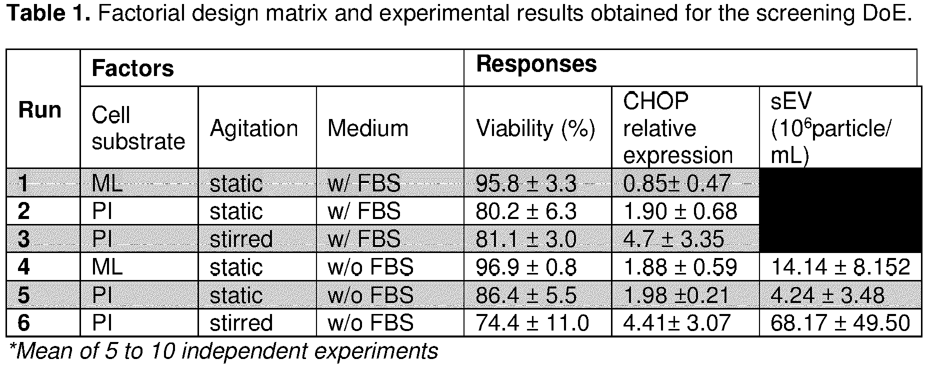

The culture of beta cells as pseudo-islets significantly decreased cell viability (p<0.05, **Table 2),** while no significant impact was observed for solely serum starvation or stirring **(Erreur ! Source du renvoi introuvable.).** By contrast, serum starvation concomitantly with stirring significantly increased the relative expression of CHOP normalized by the level of expression in beta cell monolayer cultures in medium w/FBS (p=0.0152 and 0.0043 for serum deprivation and stirring respectively, **Table 2).** Therefore, although cell viability was significantly decreased when cells were cultured as pseudo-islets, no increase of CHOP expression was observed in cells cultured (p=0.4111) suggesting that the remaining living cells were not ongoing through apoptotic pathways. The interactions of the factors cell substrate/medium and medium/agitation had no significant effect detrimental effect on beta cell viability or cellular stress. Finally, the concentration of sEV produced in the conditions of cultured w/o FBS were quantified. A significant (>4-fold) increase in the amount of sEV produced from pseudo-islets in stirred systems was observed (p<0.001, **Figure 3C****),** while no differences were observed between those produced by PI cultured in static **(****Figure 3C****).** The increase of sEV produced by pseudo-islets cultured in stirred systems could be explained by the increase of cellular stress. These results demonstrate the feasibility to produce sEV using pseudo-islets, but underlined the negative impact of stirring on cellular stress that was correlated with an increase in sEV production.

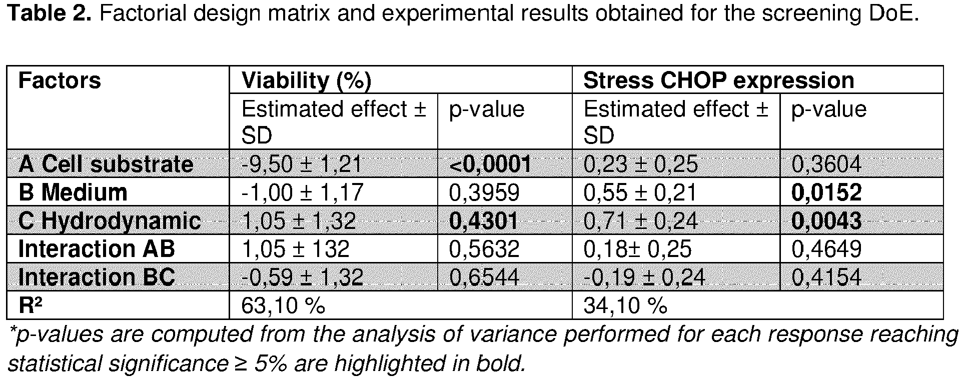

### Effect of glucose

Beta cells secrete insulin in response to glucose stimulation. In the culture media used, a high concentration of glucose is used, compared to the physiological one (25 mM versus 4.5 mM), that is known to stimulated beta cell insulin secretion. In states of increased insulin demand such as hyperglycaemia, beta cells compensate to restore glucose homeostasis via beta cell proliferation or increased insulin biosynthesis (Cerf et al., 2013). By contrast, chronic hyperglycaemia may also negatively affect beta-cell mass by inducing apoptosis without a compensatory increase in beta-cell proliferation and neogenesis. The detrimental effect of excessive glucose concentrations is referred to as "glucotoxicity". Therefore, the impact of low versus high glucose concentration was assessed during the steps of (i) beta cell amplification in monolayer culture **(****Figure 4****),** (ii) formation of pseudo-islets **(****Figure 8****Erreur ! Source du renvoi introuvable.)** and *(iii)* sEV production **(****Figure 8** and **Figure 5****).** First, the impact of glucose concentration was assessed on beta cell differentiation markers (insulin content, glucose stimulated insulin response, PDX1, NKX6.1 and INS1), cell viability (% viable cell by total cells) and cellular stress (relative expression of CHOP, NF kB and GRP94).

The culture of MIN6 beta cell line in monolayer in low glucose concentration did not modify their ability to produce insulin in response to glucose stimulation **(****Figure 4A****),** although their insulin content (p<0.005, **Figure 4B****)** and the level of the transcription factor PDX1 expressed (p<0.05, **Figure 4C****)** significantly dropped. In parallel the population doubling time of MIN6 cells cultured in low glucose concentration increased from 41.0±3.8 to 68.6±5.2 hours (p<0.005, **Figure 4D****).** These results underlined the benefit of high glucose concentration to promote beta cell proliferation and differentiation. Interestingly, the high glucose concentration was not associated to an increase of expression of the endoplasmic reticulum stress marker CHOP, which proofed lower than in beta cells cultured at low glucose concentration (p<0.005, **Figure 4F****).** The expression of other markers of stress-signalling pathways remained unchanged **Figure 4F****).**

Although a high glucose concentration of 4.5 g/L significantly improved beta cell maturation and proliferation of MIN6 cultured as adherent monolayers without increasing endoplasmic cellular stress, no impact of glucose concentration was observed on MIN6 beta cells cultured as pseudo-islets in medium w/FBS **(****Figure 8****)** or w/o FBS **(****Figure 9****).** A plausible explanation could be the increase of beta cell maturation already induced by 3D culture as well as the lower viability measured in stirred tanks that may mask the impact of glucose concentration. Finally, the yield and insulin load of sEV produced by MIN6 pseudo-islets cells cultured in stirred tanks at low or high glucose concentration were quantified **(****Figure 5****).** While the overall yield of sEV particles per mL of culture supernatant remained unchanged, production at low glucose concentration significantly enhanced export of insulin inside beta sEV (p<0.001). As EV mirror the parental cell metabolism, the glucose concentration in medium could modify the insulin content embarked in EV. As numerous targets of the T1D autoimmune response enter sEV biogenesis pathways, adjustment of medium glucose concentration during the production phase could be an indirect means to control beta sEV antigen content for immune intervention.

### Process optimization to produce sEV from mature beta cells at large-scale.

As stirring is required to easily scale up the process in bioreactors, we carried out a complete factorial plan aiming to identify and optimize the culture parameters and their interactions impacting (i) the parental cell viability, (ii) the relative expression of CHOP and (iii) sEV concentration. Three process parameters were studied: the cell seeding density (1, 2.5 or 5×10⁶ cells/mL), the speed of stirring (60, 90 or 120 rpm) and the duration of culture (4h, 24h and 44h) **(Table 3** and **Figure 6A****).**

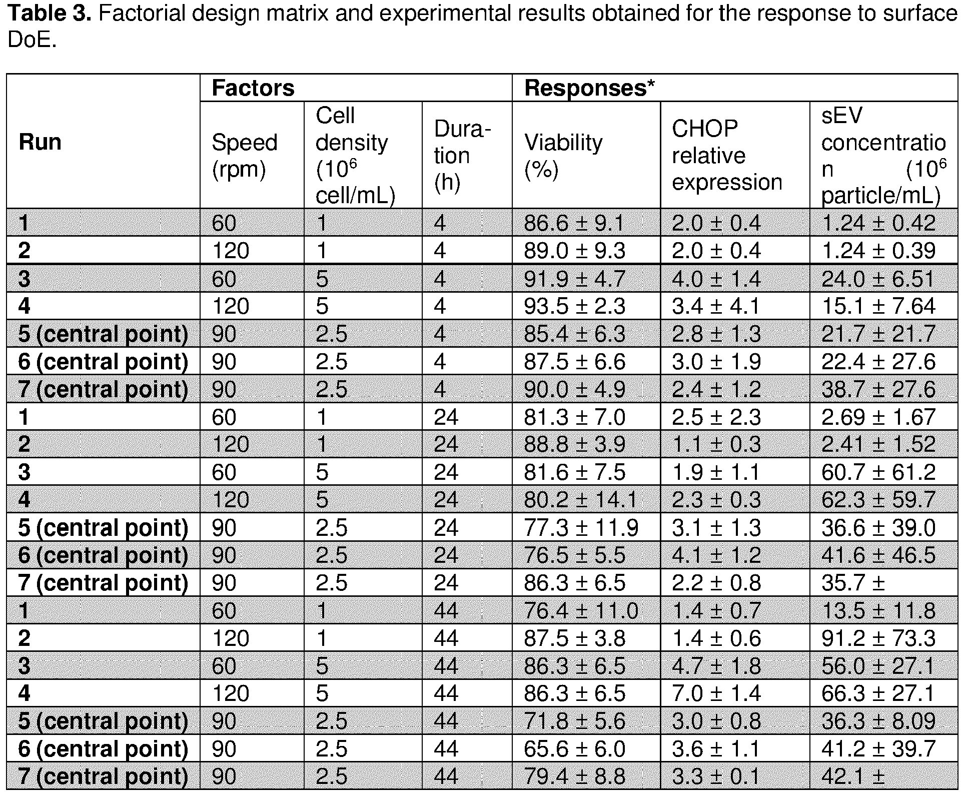

Analysis of variance showed that the duration of culture (p<0.0001 and p=0.0031), the concentration of cells (p=0.0397 and p=0.001) seeded and the interaction between these two factors (p=0.0002 and p=0.0011) significantly affects cell viability and CHOP relative expression respectively, whereas the speed of agitation has no noticeable effect **(****Figure 6** and Table). As a general rule, for a turbulent vortex to cause local dissipation of its energy triggering cell death, the Kolmogoriv scale must be smaller than or similar to the size of the cells. In this study, pseudo-islets had a diameter of around 50 µm and were stirred at 60, 90 or 120 rpm, representing a Kolmogoriv scale of up to 100 µm (Ghasemian et al., 2020). It can be assumed that stirring should not cause cell death in these settings. Furthermore, high shear stress can lead to loss of membrane integrity and contamination of the final product by non-vesicular material. This is not the case of sEV produced by high density cultures at 120 rpm for 4h as confirmed by cryo-electron microscopy analysis showing membrane vesicles with conserved morphology. Increasing the concentration of seeded cells significantly increases the concentration of sEV produced per mL of culture **(****Figure 6B****).**

Prolonged culture duration allowed increasing the sEV yield concentration solely at low seeding density. A plausible explanation for the absence of increased sEV yield over time at high cell density may be a significant reabsorption of sEV by viable cells. In line with these observations, a study proposed to monitor cell viability by sEV sensing: no sEV accumulation was measured during the exponential cell growth phase, whereas it slowly increased during the stationary phase and a lot concomitantly to cell death (Zavec et al., 2016). The stress priming method commonly used to trigger sEV production is generally paralleled by a decrease in cell viability. Thus, in addition to increasing the specific rate of sEV production under stress, cell mortality may also contribute to an apparent increase in sEV concentration by decreasing the amount of sEV reabsorbed. As the goal is to produce "healthy and mature" beta cell sEVs. Based on these results, it appears to be relevant to produce these sEVs at high cell density and over short periods of culture while ensuring a large amount of EV produced.

It could also be relevant to define a strategy of continuous or discontinuous media perfusion in order to avoid the reabsorption of sEV during the production phase.

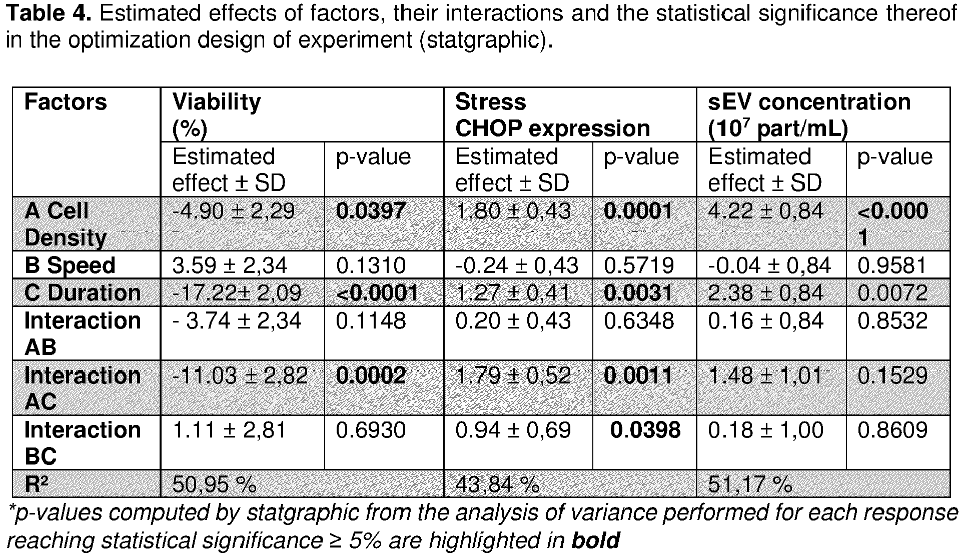

### Downstream challenges to produce therapeutic sEV.

sEV are traditionally obtained by successive centrifugation steps at increasing speed (300, 2,000 and 10,000 g) followed by an ultracentrifugation (UC) step to pellet the EVs at 100,000 g. The inventors (Bosch et al., 2016) and others (Théry et al, 2006 ; Linares et al., 2015) have shown earlier that the UC's step damages the vesicles, leads to aggregation and fusion **(****Figure 7A****)** and low sEV recovery yield. Furthermore, this workflow does not suit large scale production of sEV (manual work required, duration of the procedure, cost of equipment). Alternative precipitation techniques isolate sEV of very low purity unsuitable for clinical application. The inventors have developed a process including a primary clarification by centrifugation, a concentration by ultrafiltration (UF-100 kDa MWCO) and a polishing step based on size exclusion chromatography (SEC) that improved 38-fold particles' purity **(****Figure 7B****Erreur ! Source du renvoi introuvable.).** However, while the UF-SEC process leads to an acceptable purity ratio (Webber et al., 2013), the number of sEV recovered per seeding cells dropped down to 22.83 particles compared to precipitation yielding to 27.26 particles per cell.

The inventors hypothesized that sEV aggregation and membrane clogging during the frontal UF filtration step may strongly impact the sEV yield.

A Tangential Flow Filtration process with a 300 kDa MWCO was assessed in concentration and diafiltration mode leading to a higher yield of 47.45 particles recovered per cell, but with a low purity comparable to that obtained with sEV precipitation reagents **(****Figure 7B****).** The addition of a final polishing step using SEC or a bind and elute-size exclusion low pressure chromatography (BE-SEC) allows to obtain a similar purity ratio than for the UF-SEC process. The BE-SEC enabled on line measurement of DO and conductivity making the process more reproducible (Corso et al., 2017). than separation on columns driven only by gravity as for SEC. The very high recovery yields of particles per cell obtained by this process is encouraging preliminary results for the development of a scalable automated purification process combining TFF and a BE-SEC methods. Furthermore, implementing filtration techniques instead of centrifugation for primary clarification is also possible (Besnard et al., 2016). Normal Flow Filtration (NFF) using depth and membrane filters in a single-use format becomes a popular approach for the clarification of viruses such as rAAV. There are several types of depth filters available to accommodate process development at commercial manufacturing scales. They are often multilayered, with a nominal size of 0.1-60 µm. As evidenced by commercial processes for the purification of viral particles, the combination of depth filtration, tangential flow filtration and low pressure chromatography seems suitable for the automated, scalable and consistent downstream processing of sEV isolation from conditioned culture medium (Colao et al., 2018).

### Influence of glucose concentration

The influence of glucose concentration on MIN6 pseudo-islets culture in medium w/FBS as assayed. MIN6 cells were cultured for 3 days in stirred tank (90 rpm) with DMEM + 10% FBS with high or low glucose concentrations controlled between 4.5 and 3.5 g/L or 1.5 and 1 g/L respectively. Glucose was daily assessed (Bioprofil) and refreshed as required to reach the targeted ranges. The cells were analyzed at day 3. The results are shown on **Figure 8****.**

The influence of glucose concentration on MIN6 pseudo-islets culture in medium w/o FBS for sEV production step was assayed. MIN6 cells were cultured for 1 day in stirred tank (90 rpm) with Optimem with high or low glucose concentrations controlled between 4.5 and 3.5 g/L or 2.5 and 2 g/L respectively. The cells were analyzed at day 1. The results are shown on **Figure 9****.**

### Conclusion.

EV released from healthy mature beta cells have been shown to contribute to beta function and immune homeostasis offering new leverage for immune intervention for T1D. Given the important quantities of GMP-grade EV required for clinical application, the possibility to produce beta sEV from beta cells grown as aggregates in scalable stirred tank bioreactor manufacturing process appears as a promising option.

### REFERENCES

Besnard, L. et al. Clarification of vaccines: An overview of filter based technology trends and best practices. Biotechnol Adv 34, 1-13 (2016).
Bosch, S. et al. Trehalose prevents aggregation of exosomes and cryodamage. Scientific Reports 6, 36162 (2016).
Cerf et al. Beta cell dysfunction and insulin resistance. Front Endocrinol (Lausanne). 27;4:37 (2013).
Cianciaruso et al., Primary Human and Rat β-Cells Release the Intracellular Autoantigens GAD65, IA-2, and Proinsulin in Exosomes Together With Cytokine-Induced Enhancers of Immunity. Diabetes ;66(2):460-473 (2017)
Colao, I. L., Corteling, R., Bracewell, D. & Wall, I. Manufacturing Exosomes: A Promising Therapeutic Platform. Trends in Molecular Medicine 24, 242-256 (2018).
Corso et al. Reproducible and scalable purification of extracellular vesicles using combined bind-elute and size exclusion chromatography. Scientific Reports 7, 11561 (2017).
Ghasemian, M. et al. Hydrodynamic characterization within a spinner flask and a rotary wall vessel for stem cell culture. Biochemical Engineering Journal 157, 107533 (2020).
Giri, K. R. et al. Molecular and Functional Diversity of Distinct Subpopulations of the Stressed Insulin-Secreting Cell's Vesiculome. Front Immunol 11, (2020).
Gudbergsson, J. M., Johnsen, K. B., Skov, M. N. & Duroux, M. Systematic review of factors influencing extracellular vesicle yield from cell cultures. Cytotechnology 68, 579-592 (2016).
Hassouna et al. Functional Maturation and In Vitro Differentiation of Neonatal Porcine Islet Grafts. Transplantation. 102(10):e413-e4232018 (2018).
Herrmann et al. Extracellular vesicles as a next-generation drug delivery platform. Nature Nanotechnology. 16(7):748-759 (2021).
Hogrebe et al. Generation of insulin-producing pancreatic β cells from multiple human stem cell lines. Nature Protocols. 16(9):4109-4143 (2021).
Javeed, N. et al. Pro-inflammatory β cell small extracellular vesicles induce β cell failure through activation of the CXCL10/CXCR3 axis in diabetes. Cell Reports 36, 109613 (2021).
Linares, R., Tan, S., Gounou, C., Arraud, N. & Brisson, A. R. High-speed centrifugation induces aggregation of extracellular vesicles. J Extracell Vesicles 4, (2015).
Mastronarde, D. N. Dual-Axis Tomography: An Approach with Alignment Methods That Preserve Resolution. Journal of Structural Biology 120, 343-352 (1997).
McCluskey et al. Development and functional characterization of insulin-releasing human pancreatic beta cell lines produced by electrofusion. The Journal of Biological Chemistry, 22, 286(25):21982-21992 (2011).
Oslowski, C. M. & Urano, F. Measuring ER Stress and the Unfolded Protein Response Using Mammalian Tissue Culture System. in Methods in Enzymology vol. 490 71-92 (Elsevier, 2011).
Piffoux, M. et al. Extracellular vesicles for personalized medicine: The input of physically triggered production, loading and theranostic properties. Advanced Drug Delivery Reviews 138, 247-258 (2019).
Ravassard et al., A genetically engineered human pancreatic β cell line exhibiting glucose-inducible insulin secretion. J Clin Invest. 121(9):3589-3597 (2011).
Théry, C., Amigorena, S., Raposo, G. & Clayton, A. Isolation and characterization of exosomes from cell culture supernatants and biological fluids. Curr Protoc Cell Biol Chapter 3, Unit 3.22 (2006).
Webber et al. How pure are your vesicles? J Extracell Vesicles 2, (2013).
Yuan et al. A small-molecule inducer of PDX1 expression identified by high-throughput screening. Chem Biol. 20(12):1513-22 (2013)
Yung et al. Sufu- and Spop-mediated downregulation of Hedgehog signaling promotes beta cell differentiation through organ-specific niche signals. Nat Commun 11;10(1):46472019 (2019).
Zavec, A. B. et al. Extracellular vesicles concentration is a promising and important parameter for industrial bioprocess monitoring. Biotechnol J 11, 603-609 (2016).

## Claims

1. A method for obtaining extracellular vesicles from beta cells comprising at least a step (i) of culturing beta cells in the form of beta cell aggregates in suspension in a cell culture medium under stirring conditions to obtain extracellular vesicles from the beta cells.

2. The method of claim 1, wherein the beta cell aggregates produce and secrete extracellular vesicles in the cell culture medium.

3. The method according to any of claim 1 or 2, wherein the cell culture medium is a serum-free medium.

4. The method according to any of claims 1 to 3, wherein step (i) is performed during a period allowing to maintain a cell viability higher than 90%, for example between 2 hours and 50 hours, preferably between 4 and 24 hours.

5. The method according to any one of claims 1 to 4, wherein step (i) is performed at a stirring speed of between 60 and 160 rpm, preferably between 90 and 120 rpm.

6. The method according to any of claims 1 to 5, wherein the beta cell aggregates are pseudo-islets.

7. The method according to claim 6, wherein step (i) is preceded by a step (i₀) of forming the pseudo-islets comprising seeding a cell culture medium with isolated beta cells.

8. The method according to claim 7, wherein the cell culture medium is seeded at a cell density of between 0.3×10⁶ and 10⁷ cells per mL of cell culture medium.

9. The method according to any of claims 6 to 7, wherein the beta cells are pancreatic continuous cell line derived beta cells, stem-cell derived beta cells or primary beta cells.

10. The method according to claim 9 wherein the pancreatic continuous cell line is selected from 1.4E7, PANC-1, 1.1B4, 1.1E7 or EndoC-βH1 cell lines.

11. The method according to any of claims 1 to 10 wherein step (i) and/or step (i₀) is performed in a stirred tank bioreactor.

12. The method according to any of claim 1 to 11, wherein in step (i), culturing the beta cell aggregates is not associated to an increase of expression of markers associated with beta cells stress such as CHOP, spliced XBP1, ATF4, ATF3, NF -kB or GRP94.

13. The method according to any of claims, wherein the method further comprises a step (ii) of isolating small extracellular vesicles having a size ranging between 20 and 150 nm from the extracellular vesicles obtained at the end of step (i).

14. The method according to claim 13, wherein step (ii) comprises the steps of:
(iia) a primary clarification by centrifugation or depth filtration, preferably by normal flow filtration (NFF),
(iib) a concentration by ultracentrifugation or Tangential Flow Filtration (TFF), and
(iic) a filtration by low pressure chromatography, preferably by size exclusion chromatography (SEC) or by bind and elute-size exclusion low pressure chromatography (BE-SEC).

15. The extracellular vesicles, obtainable by the method according to any of claims 1 to 14, in particular the small extracellular vesicles having a size ranging between 20 and 150 nm obtainable by the method according to any of claims 13 to 14.
